# EUROPEAN PATENT SPECIFICATION

(11) **EP 2 581 745 B1**
(45) Date of publication and mention of the grant of the patent: **30.12.2015**
(21) Application number: 12150252.0
(22) Date of filing: 05.01.2012
(51) Int. Cl.: G01N 33/574

(54) **Composition for diagnosis of lung cancer and diagnosis kit of lung cancer**
Zusammensetzung für die Diagnose von Lungenkrebs und Diagnosekit für Lungenkrebs
Composition pour le diagnostic du cancer des poumons et kit de diagnostic du cancer des poumons

(30) Priority: 14.10.2011 KR 20110104949
(43) Date of publication of application: 17.04.2013
(73) Proprietor: Seoul National University R&DB Foundation, Seoul 151-742 (KR)
(72) Inventor: Cho, Je Yoel, Seoul 135-506 (KR); Sung, Hye Jin, Gyeongsangbuk-do 770-842 (KR)
(74) Representative: Peter, Julian

(56) References cited:
- WO-A1-2010/071788
- WO-A1-2010/086380
- WO-A1-2010/086384

## Description

### BACKGROUND OF THE INVENTION

### 1. Field of the invention

The present invention relates to the use of a composition for diagnosis of lung cancer, and a lung cancer diagnosis method using the same, in which the composition includes a lung cancer-specific protein marker whose expression is increased or decreased in a lung cancer tissue.

### 2. Description of the Prior Art

Lung cancer is currently one of the cancers showing high lethality overseas as well as domestically. Such a tendency is caused by limited subjective symptoms, and the absence of an early diagnosis method having a high sensitivity. At present, the diagnosis of lung cancer relatively highly depends on an imaging method (X-ray, CT, MRI, etc.) while there are few examples of the discovery of a substance capable of being used as a biochemical indicator.

A biomarker includes all physical and biochemical indicators capable of diagnosing the physiological and pathological states of a body. At present, the paradigm of cancer treatment is changing from the development of an antitumor agent for directly treating a cancer to induction of early detection, and effective treatment and continuous monitoring based on the detection. Accordingly, the most required thing for this change is a biomarker, that is, a substance for early diagnosing and monitoring of a cancer.

There are some substances, called biomarkers, which are suggested for cancer diagnosis. However, they have not yet sufficiently shown specificity and sensitivity. Thus, in various diseases, cross-reactivity exists, while there is no method for complementing this. For this reason, even in the USA, there are few actually clinically applied biomarkers with FDA approval.

In a case of lung cancer, many theses on a biomarker candidate substance have been published, and research on novel biomarker candidate proteins is continuously being conducted. However, up to now, there is no commercialized biomarker capable of specifically diagnosing lung cancer.

A body fluid directly reflects various kinds of disease states, and thus is considered to store the records on a change of a body. In other words, the body fluid is an important factor in early diagnosis and prediction of a disease.

For this reason, research on finding a disease-specific protein by using a body fluid (such as serum and cerebrospinal fluid) capable of easily being obtained and handled has been continuously conducted. The representative body fluid, most widely used for diagnosis, is blood. However, blood exists farthest away from a tissue origin. While being secreted, the blood moves away from the origin. Thus, it is difficult to find a tissue-specific origin protein, and also there exist many difficulties in detection due to a wide dynamic range of a blood protein. Accordingly, for discovery of a tissue-specific cancer biomarker, a currently widely used method, that is, through a LC-MS/MS analysis using a blood protein, accompanies many difficulties. In consideration of such difficulties, it is determined that finding a biomarker directly from a tissue is advantageous in the discovery of a tissue/individual specific biomarker according to the kind of cancer.

Prior art documents also cover WO2010/071788 A1, WO2010/086384 A1 and WO2010/086380 A1.

### SUMMARY OF THE INVENTION

In the present invention, through direct discovery of a lung cancer-specific biomarker from a tissue, lung cancer can be quickly, simply, and accurately diagnosed.

The present invention provides the use of a composition, including an antibody specifically binding to Quiescin-sulfhydryl oxidase 1 in the in vitro/ex vivo diagnosis of lung cancer.

The present invention provides the use of a composition including a primer or a probe specific to a nucleic acid encoding at least Quiescin-sulfhydryl oxidase 1 in the in vitro/ex vivo diagnosis of lung cancer.

In the present invention, the lung cancer is at least one selected from the group including lung adenocarcinoma, squamous cell carcinoma, large cell cancer, and small cell cancer.

The present invention provides the use of a diagnostic kit including the composition as an active ingredient in the in vitro/ex vivo diagnosis of lung cancer.

The present invention provides a lung cancer diagnostic microarray including the composition as an active ingredient.

The present invention provides a method for detecting, from a biological sample, Quiescin-sulfhydryl oxidase 1 as a lung cancer marker.

In the present invention, the biological sample is at least one selected from the group including tissues, cells, whole blood, serum, plasma, saliva, sputum, and urine, obtained from mammals.

The inventive composition for diagnosis of lung cancer is excellent in sensitivity and specificity, and thus can be usefully used in early diagnosis of lung cancer.

### BRIEF DESCRIPTION OF THE DRAWINGS

The above and other objects, features and advantages of the present invention will be more apparent from the following detailed description taken in conjunction with the accompanying drawings, in which:
FIG. 1 shows primary culture of cells separated from a normal surrounding tissue and a lung cancer tissue of a lung cancer patient;
FIG. 2 shows SDS-PAGE separation of proteins from a normal surrounding tissue and a lung cancer tissue of a lung cancer patient, and coomassie staining of them;
FIG. 3 shows LC-MS/MS analysis of Quiescin-sulfhydryl oxidase 1 (QSOX1);
FIG. 4 shows the comparison between a normal cell line and a lung cancer cell line in mRNA expression and protein expression of Quiescin-sulfhydryl oxidase 1 (QSOX1); and
FIG. 5 shows the comparison between a normal surrounding tissue and a lung cancer tissue in protein expression of Quiescin-sulfhydryl oxidase 1 (QSOX1) of lung cancer patients.

### DETAILED DESCRIPTION OF THE PREFERRED EMBODIMENTS

The present invention provides the use of a composition for diagnosis of lung cancer, which includes an antibody specifically binding to Quiescin-sulfhydryl oxidase 1 Preferably, the composition for diagnosis of lung cancer includes an antibody specifically binding to the marker, as an active ingredient.

In the present invention, the term "diagnosis" indicates determination of existence or characteristics of pathologies. In view of the objective of the present invention, the diagnosis is on the onset of lung cancer.

In the present invention, the term "lung cancer" means a malignant tumor occurring in a lung, and histologically includes lung adenocarcinoma, squamous cell carcinoma, large cell lung cancer, and small cell lung cancer.

Lung cancer has 4 main histological types, and then is further subdivided into two types of small cell lung cancer (SCLC) and non-small cell lung cancer (NSCLC). The non-small cell lung cancer (NSCLC) is found in about 80% of all lung cancer patients, and includes lung adenocarcinoma, squamous cell carcinoma, and large cell lung cancer. From among some histological types of NSCLC, squamous cell carcinoma is found in 20-25% of lung cancer patients, and large cell lung cancer is found in 15-20%, and lung adenocarcinoma is found in 30-40%.

In the present invention, an antibody may be a whole form of antibody (hereinafter, referred to as a "whole antibody") or its functional fragment. The whole antibody may be a polymer type of antibody including monomers or 2 or more bound whole antibodies. The functional fragment of the antibody is an antibody having a heavy chain variable region and a light chain variable region of the whole antibody, and in actuality, recognizes the same epitope as that recognized by the whole antibody.

The functional fragment of the antibody includes single chain variable region fragments (scFv), (scFv)2, Fab, Fab', F(ab')2, and the like, but the present invention is not limited thereto. The single chain variable region (scFv) indicates a single chain polypeptide antibody fragment in which a heavy chain variable region and a light chain variable region are linked by a linker peptide.

The antibody may be modified by attachment with various molecules such as an enzyme, a fluorescent material, a radioactive material and a protein. The modified antibody may be obtained by chemically modifying the antibody. This modification method is conventionally used in the art. Also, the antibody may be obtained as a chimeric antibody having a variable region derived from a non-human antibody, and a constant region derived from a human antibody, or may be obtained as a humanized antibody including a complementarity-determining region derived from a non-human antibody, and a framework region (FR) and a constant region derived from a human antibody. Such an antibody may be prepared by using a method known in the art.

The diagnostic composition may further include a reagent used for immunoassay, known in the art, besides the protein-specific antibody. The immunoassay may include any method as long as it can measure binding between an antigen and an antibody.

Also, the present invention provides the use of a diagnostic kit of lung cancer, which includes the composition as an active ingredient.

The diagnostic kit may be, for example, an immunochromarography-based lateral flow assay kit for detecting a specific protein from a test sample. The lateral flow assay kit generally includes a sample pad to which a test sample is applied, a releasing pad coated with a detection antibody, a transfer membrane (e.g., nitrocellulose) or a strip in which the test sample is moved and separated, and an antigen-antibody reaction occurs, and an absorption pad.

Also, the present invention provides the use of a diagnostic microarray of lung cancer, which includes the composition as an active ingredient.

In the microarray, in general, an antibody is immobilized on a slide glass surface treated with a specific reagent, and then a protein specifically bound to the antibody through an antigen-antibody reaction is detected.

Also, the diagnostic composition of lung cancer includes, as an active ingredient, a primer or a probe specific to a nucleic acid encoding at least Quiescin-sulfhydryl oxidase 1.

The detection of a specific nucleic acid by using the primer may be carried out by amplifying a sequence of a target gene through an amplification method such as PCR, and detecting the amplification of the gene according to a method known in the art. Also, the detection of a specific nucleic acid by using the probe may be carried out by contacting a test sample nucleic acid with the probe under a proper condition, and detecting the existence of a hybridized nucleic acid.

The term "primer" indicates a short nucleic acid sequence having short free OH groups, which can form a base pair with a complementary template, and functions as a start point for template strand replication.

The "probe" indicates a fragment of a nucleic acid including several to several hundreds of bases (such as RNA or DNA) capable of being specifically bound to mRNA. The probe is labeled and thus can be used for determining the existence of a specific mRNA. The probe may be produced as various types of probes such as an oligonucleotide probe, a single stranded DNA probe, double stranded DNA probe, a RNA probe, and may be labeled with biotin, FITC, rhodamine, DIG, radio isotope, or the like.

Also, the probe may be labeled with a detectable substance, for example, a radio label capable of providing an appropriate signal and having a sufficient half life. The labeled probe may be hybridized with a nucleic acid on a solid substrate, as known in the art.

Examples of the method for detecting a specific nucleic acid by using the probe or the primer may include, but are not limited to, polymerase chain reaction (PCR), DNA sequencing, RT-PCR, primer extension, oligonucleotide extension analysis, allele-specific PCR, RNase mismatch cleavage, single strand conformation polymorphism (SSCP) and heteroduplex simultaneous analysis, denaturing gradient gel electrophoresis (DGGE), denaturing high pressure liquid chromatography, hybridization reaction, DNA chip, and the like. Examples of the hybridization reaction include northern hybridization, in situ hybridization, and microarray.

The composition for diagnosis of lung cancer may further include a conventionally used reagent in the above described nucleic acid detecting method. For example, it may include a metal ion salt required for a PCR reaction, such as dNTP (deoxynulceotide triphosphate),

thermostable polymerase, and magnesium chloride, and include dNTP, sequenase, etc. required for sequencing.

Preferably, the composition for diagnosis of lung cancer may be provided in the form of a diagnostic kit or microarray. For example, the inventive composition may be provided as a RT-PCR kit, a DNA chip, or the like, but the present invention is not limited thereto, in which the RT~PCR kit includes respective primer pairs specific to at Quiescin-sulfhydryl oxidase 1.

Also, the present invention provides a method for detecting, from a biological sample, Quiescin-sulfhydryl oxidase 1 as a lung cancer marker through an antigen-antibody binding reaction using an antibody specifically bound to Quiescin-sulfhydryl oxidase 1.

In the present invention, through an antigen-antibody binding reaction using an antibody specifically bound to Quiescin-sulfhydryl oxidase 1 as a lung cancer marker, the protein is detected from a biological sample so as to predict the diagnosis or prognosis of lung cancer early. Specifically, the proteins are fractionated through electrophoresis (SDS-PAGE), transferred to a solid support, and immobilized. An antibody specifically bound to the proteins is added to perform an antigen-antibody binding reaction, and then expression levels of these proteins are measured. In other words, from a lung cancer patient tissue and a healthy person tissue, the expression levels of the proteins are measured, and compared to each other. Then, when the expression level of the protein in the lung cancer patient
tissue is higher than that in the healthy person, he is diagnosed to have lung cancer or expected to have lung cancer.

Examples of the biological sample may include test samples obtained from mammals, such as tissues, cells, whole blood, serum, plasma, saliva, sputum, and urine, but the present invention is not limited thereto. The test sample may be subjected to a post-treatment such as filtration, distillation, extraction, concentration, inactivation of an interrupting substance, or addition of a reagent, before being used for the detection.

As a solid support for the antigen-antibody binding reaction, a nitrocellulose membrane, a PVDF membrane (polyvinylidene difluoride membrane), a 96 well plate synthesized by a polyvinyl resin or a polystyrene resin, or a slide glass made of a glass may be used.

The antigen-antibody binding reaction may be carried out by a conventional method such as enzyme-linked immunosorbent assay (ELISA), radioimmunoassay (RIA), sandwich assay, western blotting, immunoprecipitation, immunohistochemical staining, flow cytometry, fluorescence activated cell sorter (FACS), chromogenic enzyme substrate test, and antigen-antibody aggregation.

Hereinafter, a preferred embodiment will be provided for illustrating the present invention. However, the Examples below are intended to further illustrate the present invention without limiting its scope.

### <Example 1> Test sample collection

A system for primary culture using a lung cancer tissue and a surrounding normal tissue obtained from a lung cancer patient was secured. The lung cancer tissue and the surrounding normal tissue were maintained in a fresh state, and were chopped by using scissors or a blade in accordance with a mechanical method. Then, they were treated with collagenase type 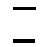 , reacted for 30 minutes to 90 minutes at 37°C so as to separate single cells. Then, they were plated onto a medium containing 10% FBS, and cultured.

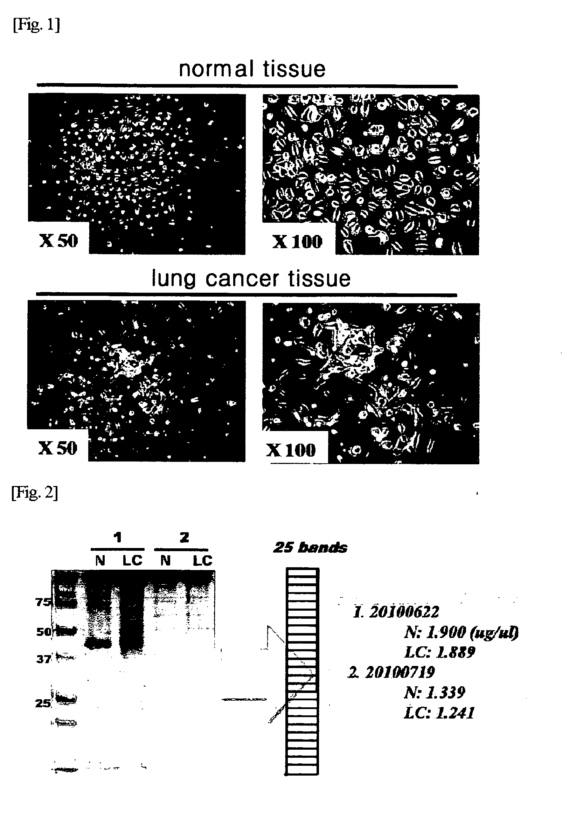

In a case of a cell separated from the normal tissue, a typical epithelial cell type of pebble-shaped circular cells were grown, while in a case of a cell separated from the lung cancer tissue, fibrocyte-like shaped cells were grown in a long form (see FIG. 1).

### <Example 2> Separation and concentration of a secreted protein

Cells from Example 1 were subcultured twice to three times so as to collect a protein secreted during a culturing process. In subculture 3, the same number of cells were counted, and they were transferred to a new culturing plate, and cultured for 48 hours to collect a medium including a secreted protein.

Then, in order to remove the FBS content contained in a subculture medium of the normal cell plate and the lung cancer cell plate, each plate was sufficiently washed. Then, the medium was replaced by a serum free media, and culturing was performed for 24 to 48 hours. In this process, in order to obtain a protein secreted from each cell, a TCA precipitation method was used for protein concentration.

### <Example 3> Separation and coomassie blue staining of SDS-PAGE

The protein from Example 2, which was secreted from the normal tissue cell and the lung cancer tissue cell and concentrated, was separated through SDS-PAGE, and the gel was washed with ddH2O three times (each for 5 minutes). Then, the protein was added with Coomassie G250 Stain (BioRad, Hercules, CA), and softly shaken at room temperature for 1 hour for staining. In the coomassie staining, in each pair (N: a protein secreted from a normal tissue, LC: a protein secreted from a lung cancer tissue), the proteins were used for the experiment in the same amount (see FIG. 2).

### <Example 4> In-gel digestion

After the staining in Example 3, an interesting protein band was cut into pieces, and was subjected to in-gel trypsin digestion. 25 bands showing different
respective strengths of the protein secreted from the lung cancer tissue, and the protein secreted from the normal tissue were analyzed.

### <Example 5> Semi-quantitative analysis on a secreted protein

In semi-quantitative proteomics, without using a labeling material, a spectral count (that is, the number of peptides, obtained during analysis for protein identification after MS/MS analysis), is used. The secreted protein was analyzed by a conventional semi-quantitative method, and the identified protein was subjected to gene ontology analysis so as to determine the characteristics of proteins obtained from the experimental result.

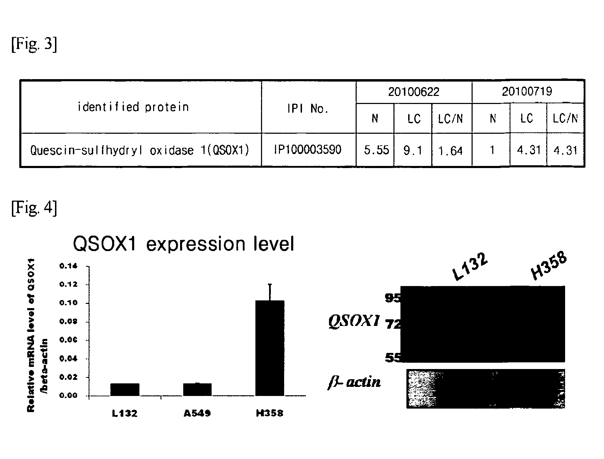

Through LC-ESI-MS/MS analysis, from two pairs (20100622, 20100719) of samples, a protein of Quiescin-sulfhydryl oxidase 1 (QSOX1) was found. In respective samples, the protein secreted from the lung cancer tissue (LC) showed 1.64 and 4.31 times higher quantitative values, respectively, compared to the protein secreted from the normal tissue (N) (see FIG. 3).

**[Table 1]**

| Identified protein | IPI number | clone: 20100622 | | | clone: 20100719 | | | Average value. lung cancer/ normal |
|---|---|---|---|---|---|---|---|---|
| | | quantitative value | | | quantitative value | | | |
| | | normal | Lung cancer | Lung cancer/normal | normal | Lung cancer | Lung cancer/normal | |
| Fibrillin-1 | IPI00328113 | 5.55 | 74.58 | 13.44 | 1 | 4.31 | 4.31 | 8.87 |
| Isoform A of Lamin-A/C | IPI00021405 (+2) | 4.44 | 18.19 | 4.097 | 1 | 3.44 | 3.44 | 3.77 |
| Latent-transforming growth factor beta-binding protein 2 | IPI00292150 | 3.33 | 13.64 | 4.096 | 1 | 2.58 | 2.58 | 3.34 |
| Galectin-1 | IPI00219219 | 4.44 | 12.73 | 2.867 | 1 | 3.44 | 3.44 | 3.15 |
| highly similar to Dickkopf-related protein 3 | IPI00002714 (+2) | 7.77 | 20.92 | 2.692 | 1 | 5.17 | 5.17 | 3.93 |
| Isoform A1-B of Heterogeneous nuclear ribonucleoprotein A1 | IPI00215965 (+3) | 2.22 | 5.46 | 2.459 | 1 | 1.72 | 1.72 | 2.09 |
| 14-3-3 protein epsilon | IPI00000816 | 8.88 | 20.92 | 2.356 | 4.77 | 7.75 | 1.625 | 1.99 |
| Stanniocalcin-2 | IPI00008780 | 12.21 | 24.56 | 2.011 | 1 | 9.47 | 9.47 | 5.74 |
| Cystatin-C | IPI00032293 | 8.88 | 17.28 | 1.946 | 3.58 | 6.89 | 1.925 | 1.94 |
| Isoform 1 of Connective tissue growth factor | IPI00020977 | 6.66 | 11.82 | 1.775 | 1 | 5.17 | 5.17 | 3.47 |
| Profilin-1 | IPI00216691 | 8.88 | 15.46 | 1.741 | 2.38 | 6.89 | 2.895 | 2.32 |
| Isoform 1 of Extracellular matrix protein 1 | IPI00003351 (+1) | 23.32 | 39.11 | 1.677 | 11.92 | 18.08 | 1.517 | 1.60 |
| Unknown | ##IPI00008556 | 4.44 | 7.28 | 1.64 | 1 | 3.44 | 3.44 | 2.54 |
| Histone H2B type 2-E | IPI00003935 (+9) | 3.33 | 5.46 | 1.64 | 1 | 2.58 | 2.58 | 2.11 |

Also, through LC-ESI-MS/MS analysis, besides Quiescin-sulfhydryl oxidase 1 (QSOX1), other proteins whose secretions were increased in the lung cancer tissue compared to in the normal tissue were found. The result is noted in table 1. Meanwhile, through LC-ESI-MS/MS analysis, some proteins whose secretions were decreased in the lung cancer tissue compared to in the normal tissue were found. The result is noted in table 2.

**[Table 2]**

| Identified protein | IPI number | clone: 20100622 | | | clone: 20100719 | | | Average value. lung cancer/ normal |
|---|---|---|---|---|---|---|---|---|
| | | quantitative value | | | quantitative value | | | |
| | | normal | lung cancer | lung cancer /normal | normal | lung cancer | lung cancer /normal | |
| Kinesin-like protein KIF26A | IPI00788247 (+1) | 2.22 | 1 | 0.45 | 2.38 | 1.72 | 0.723 | 0.59 |
| Zinc finger protein 516 | IPI00852669 (+1) | 2.22 | 1 | 0.45 | 2.38 | 1.72 | 0.723 | 0.59 |
| Interstitial collagenase | IPI00008561 (+1) | 11.1 | 3.64 | 0.328 | 17.88 | 8.61 | 0.482 | 0.40 |
| Isoform 1 of A-kinase anchor protein 9 | IPI00019223 (+4) | 6.66 | 1 | 0.15 | 11.92 | 6.89 | 0.578 | 0.36 |

### <Example 6> Verification of a target protein in a lung cancer cell line

From a L132 cell of a cell line of a lung epithelial cell, and a H358 cell of a cell line of non-small cell lung cancer (NSCLC), mRNA and protein of Quiescin-sulfhydryl oxidase 1 were obtained, and then through real-time PCR, and western blotting analysis, the expression was verified.

As a result, it was found that expression levels of mRNA and protein of Quiescin-sulfhydryl oxidase 1 were highly increased in the H358 cell line, compared in the normal cell line (L132) (see FIG. 4).

### <Example 7> Verification of a target protein in a cancer patient tissue

In order to determine if Quiescin-sulfhydryl oxidase 1 expression verified in the lung cancer cell line occurs in a lung cancer patient, in a lung cancer tissue and a surrounding normal tissue obtained from a total of 10 patients, protein expression levels were measured through western blotting.

First, from the lung cancer tissue and the surrounding normal tissue separated from 10 lung cancer patients, a protein was separated. Simply, blood of the lung cancer tissue was washed with PBS, and the tissue was homogenized in a RIPA buffer containing a protease inhibitor.

Then, proteins of respective samples were collected in the same amount by a quantitative method through Bradford assay, and then in the normal tissue and the lung cancer tissue, the expression level of Quiescin-sulfhydryl oxidase 1 protein was measured. Since the expression levels of beta actin in the normal tissue and the lung cancer tissue are similar to each other, it can be determined that the proteins were used in the same amount in the experiment.

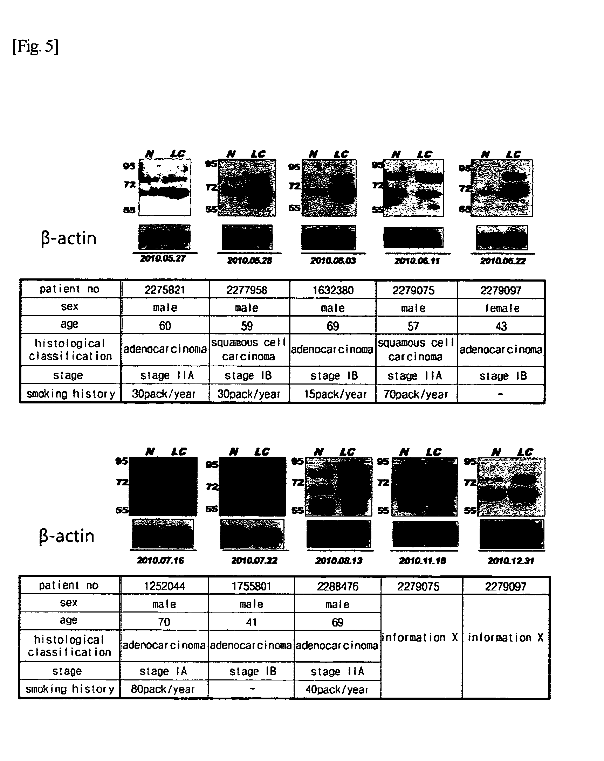

At the lower position (68kDa) and at the higher position (82kDa) with respect to the marker of 72kDa, expression levels of the secreted form and the membrane bound form were significantly increased in all lung cancer tissues of the 10 lung cancer patients. Accordingly, it can be determined that the expression level in the clinical samples was observed in the same manner as in the lung cancer cell line (see FIG. 5).

As described above, the expression level of the protein was significantly increased in the lung cancer tissue compared to that in the normal tissue. Thus, the protein can be in actuality utilized in the diagnosis of lung cancer.

Although a preferred embodiment of the present invention has been described for illustrative purposes, those skilled in the art will appreciate that various modifications, additions and substitutions are possible, without departing from the scope of the invention as disclosed in the accompanying claims.

## Claims

1. Use of a composition comprising an antibody specifically binding to Quiescin-sulfhydryl oxidase 1 in the in vitro/ex vivo diagnosis of lung cancer.

2. Use of the composition as claimed in claim 1, wherein the lung cancer is at least one selected from the group including lung adenocarcinoma, squamous cell carcinoma, large cell cancer, and small cell cancer.

3. Use of a diagnostic kit comprising the composition as claimed in claim 1 as an active ingredient in the in vitro/ex vivo diagnosis of lung cancer.

4. Use of a diagnostic microarray comprising the composition as claimed in claim 1 as an active ingredient, in the in vitro/ex vivo diagnosis of lung cancer.

5. Use of a composition comprising a primer or a probe specific to a nucleic acid encoding Quiescin-sulfhydryl oxidase 1 in the in vitro/ex vivo diagnosis of lung cancer.

6. Use of a diagnostic kit comprising the composition as claimed in claim 5 as an active ingredient in the in vitro/ex vivo diagnosis of lung cancer.

7. Use of a diagnostic microarray comprising the composition as claimed in claim 5 as an active ingredient, in the in vitro/ex vivo diagnosis of lung cancer.

8. A method for detecting, from a biological sample, Quiescin-sulfhydryl oxidase 1 as a lung cancer marker through an antigen-antibody binding reaction using an antibody specifically bound to Quiescin-sulfhydryl oxidase 1.

9. The method as claimed in claim 8, wherein the biological sample is at least one selected from the group including tissues, cells, whole blood, serum, plasma, saliva, sputum, and urine obtained from mammals.

## Patentansprüche

1. Verwendung einer Zusammensetzung, umfassend einen an Quiescin-Sulfhydryloxidase selektiv bindenden Antikörper in der in vitro / ex vivo-Diagnose von Lungenkrebs.

2. Verwendung der Zusammensetzung nach Anspruch 1, wobei der Lungenkrebs mindestens einer ist, ausgewählt aus der Gruppe einschließlich Lungenadenokarzinom, Plattenepithelkarzinom, großzelliger Kreis und kleinzelliger Krebs.

3. Verwendung eines diagnostischen Kits, umfassend die Zusammensetzung nach Anspruch 1 als Wirkstoff in der in vitro / ex vivo-Diagnose von Lungenkrebs.

4. Verwendung eines diagnostischen Mikroarrays, umfassend die Zusammensetzung nach Anspruch 1 als Wirkstoff in der in vitro / ex vivo-Diagnose von Lungenkrebs.

5. Verwendung einer Zusammensetzung, umfassend einen Primer oder eine Probe, die spezifisch für eine Quiescin-Sulfhydryloxidase 1 kodierenden Nukleinsäure ist, in der in vitro / ex vivo-Diagnose von Lungenkrebs.

6. Verwendung eines diagnostischen Kits, umfassend die Zusammensetzung nach Anspruch 5 als Wirkstoff in der in vitro / ex vivo-Diagnose von Lungenkrebs.

7. Verwendung eines diagnostischen Mikroarray, umfassend die Zusammensetzung nach Anspruch 5 als Wirkstoff in der in vitro / ex vivo-Diagnose von Lungenkrebs.

8. Verfahren zur Erkennung von Quiescin-Sulfhydryloxidase 1 als Lungenkrebs Marker aus einer biologischen Probe durch eine Antigen-Antikörper-Bindungsreaktion unter Verwendung eines an Quiescin-Sulfhydryloxidase selektiv gebundenen Antikörpers.

9. Verfahren nach Anspruch 8, wobei die biologische Probe mindestens eine ist, ausgewählt aus der Gruppe einschließlich von Säugern erhaltener Gewebe, Zellen, Vollblut, Serum, Plasma, Speichel, Sputum und Urin.

## Revendications

1. Utilisation d'une composition comprenant une mise au repos à sulfhydryle oxydase qui se lier sélectivement au anticorps dans le diagnostic in vitro / ex vivo de cancer du poumon.

2. Utilisation de la composition selon la revendication 1, dans lequel le cancer du poumon est au moins choisi du groupe y comprenant l'adénocarcinome du poumon, un carcinome à cellules squameuses, le cancer à grandes cellules et un cancer du poumon.

3. Utilisation d'un kit de diagnostic comprenant la composition selon la revendication 1 en tant qu'ingrédient actif dans le diagnostic in vitro / ex vivo de cancer du poumon.

4. Utilisation d'un puces de diagnostic comprenant la composition selon la revendication 1 en tant qu'ingrédient actif dans le diagnostic in vitro / ex vivo de cancer du poumon.

5. Utilisation d'une composition comprenant une amorce ou d'un échantillon qui est une acide nucléique spécifiquement code pour une mise au repos à sulfhydryle oxydase pour le diagnostic in vitro / ex vivo de cancer du poumon.

6. Utilisation d'un kit de diagnostic comprenant la composition selon la revendication 5 comme ingrédient actif dans le diagnostic in vitro / ex vivo de cancer du poumon.

7. Utilisation d'une microarray de diagnostic, comprenant la composition selon la revendication 5 comme ingrédient actif dans le diagnostic in vitro / ex vivo de cancer du poumon.

8. Procédé pour la détection d'une mise au repos à sulfhydryle oxydase de sulfhydryle comme marqueur du cancer du poumon d'un échantillon biologique par une réaction de liaison antigène-anticorps en utilisant un anticorps lié de manière sélective à une mise au repos à sulfhydryle oxydase.

9. Procédé selon la revendication 8, dans lequel l'échantillon biologique est au moins choisi d'un groupe y comprenant les tissus de mammifères, des cellules reçues, le sang total, le sérum, le plasma, la salive, des expectorations et l'urine.
